# EUROPÄISCHE PATENTSCHRIFT

(11) **EP 2 452 717 B1**
(45) Veröffentlichungstag und Bekanntmachung des Hinweises auf die Patenterteilung: **01.01.2014**
(21) Anmeldenummer: 11007006.7
(22) Anmeldetag: 27.08.2011
(51) Int. Cl.: A61M 16/08, A61M 16/10

(54) **Beheiztes Beatmungsschlauchsystem**
Heated breathing tube system
Système de tube respiratoire chauffé

(30) Priorität: 12.11.2010 DE 102010051079
(43) Veröffentlichungstag der Anmeldung: 16.05.2012
(73) Patentinhaber: WILAmed GmbH, 91126 Kammerstein (DE)
(72) Erfinder: Lanfranchi, Marcello, 91126 Schwabach (DE)
(74) Vertreter: Stippl, Hubert

(56) Entgegenhaltungen:
- EP-A1- 1 352 670
- EP-A1- 2 123 321
- WO-A1-97/18001
- GB-A- 1 502 113
- US-A- 4 007 737
- US-A- 5 937 855

## Beschreibung

### Technologischer Hintergrund

Der Gegenstand der vorliegenden Erfindung betrifft ein Beatmungsschlausystem zur Beatmung eines Patienten unter Atemgaskonditionierung. Atemgaskonditionierung ist eine Methode zur künstlichen Erwärmung und Befeuchtung des Atemgases bei maschinell beatmeten Patienten. Darüber hinaus kann noch eine Reinigung des Atemgases vorgenommen werden. Die Atemgaskonditionierung dient der Vorbereitung des inspirierten Atemgases (Beatmungsgas) für empfindliche Lungen. Bleibt die Atemgaskonditionierung aus, können pulmonale Infektionen und eine Schädigung des Lungengewebes die Folge sein.

Bei einer nicht-invasiven Beatmung (z.B. durch Atemmasken) wird ein kontinuierlich positiver Flow verabreicht (z.B. CPAP). Langfristig können die oberen Atemwege durch permanent druckpositive Versorgung mit zu kühlen Atemgasen austrocknen. Die Folge sind schmerzhaft entzündete Nasen- und Mundschleimhäute, Blockierungen der Luftwege sowie Sekretstau im Atmungsapparat. Durch kontinuierliche Zufuhr von körperwarmen Beatmungsgasen können diese Probleme verhindert werden.

Neugeborene sowie insbesondere Frühchen sind gegenüber derartigen Komplikationen besonders gefährdet, da sie zwar bereits überlebensfähig sind, aber ihre Lungen, der Bronchialbaum sowie die mukoziliäre Clearance noch extrem unterentwickelt ist. Um eine Austrocknung oder Verhärtung der Lunge vorzubeugen, ist eine optimale Atemgaskonditionierung bei beatmeten Frühchen und Neugeborenen zwingend notwendig.

Die Beatmung erfolgt hierbei über ein Beatmungsgerät, auch Atemgasbefeuchter genannt, mit dem das Atemgas zum einen auf den erforderlichen Feuchtegrad gebracht wird und zum anderen auf die erforderliche Temperatur erwärmt wird. Hierbei wirken das Beatmungsgerät sowie das Beatmungsschlauchsystem, welches das Beatmungsgerät mit dem Patienten verbindet, funktionell eng zusammen. Denn auch im Bereich des Beatmungsschlauchsystems wird durch eine Heizeinrichtung eine Temperierung des Atemgases bewirkt, da ansonsten ein Auskühlen entlang des Schlauches des Beatmungsschlauchsystems eintreten oder sich Kondensat bilden würde.

### Stand der Technik

Ein Beatmungsschlauchsystem gemäß dem Oberbegriff des Anspruchs 1 ist aus der EP 1 352 670 A 1 bekannt. Es ist eineinziger Heizdraht für den ersten und zweiten Schlauch vorgesehen, der in Form einer Schlaufe geführt ist und die beiden Endabschnitte des Heizdrahts verlaufen an einem T-Verbinder nach außen. Ein dritter Schlauch zum Abführen der Atemluft ist ebenfalls beheizt.

Die US 4 007 737 beschreibt ein Beatmungsschlauchsystem mit einer Membran im patientenseitigen Konnektor, die abhängig von der Richtung der Druckbeaufschlagung den Durchlass für Expirationsgas sperrt oder freigibt.

Die WO 97/18001 beschreibt ein beheiztes Beatmungsschlauchsystem mit getrennt verlaufenden, beheizten Beatmungsgas- bzw. Expirationsgasschläuchen, der einen spiralförmig verlaufenden Heizdraht beinhalten und in Form einer Schlaufe geführt sind. Am jeweiligen Ende des betreffenden Heizdrahtes ist ein außerhalb des Schlauchendes positionierte Hülse vorgesehen, in die ein elektrischer Verbinder eingesteckt werden kann.

Aus der US 4 967 744 ist ein Koaxialbeatmungsschlauchsystem gemäß dem Obergriff des Anspruch 1 bekannt. Der innere Schlauch dieses Koaxialsystems ist an seiner Außenseite mit einem Heizdraht umwickelt. Der Heizdraht befindet sich somit im äußeren Ringraum des Koaxialschlauchsystems, welcher das Expirationsgas vom Patienten wegführt. Der vorerwähnte Heizdraht wird in den abzweigenden Teils des am Beatmungsgerät befindlichen Konnektors von außen eingeführt, um etwa 180 ° gedreht und entlang der Außenseite des inneren Schlauchs geführt. Die Konstruktion ist zum einen aufwändig und daher insbesondere für eine Einweglösung ungeeignet. Zum anderen wird nur eine unzureichende Temperaturkonstanz des Inspirationsgases und damit eine insbesondere für Frühchen oder Neugeborenen unzureichende Konditionierung des Atemgases erzielt.

Aus der EP 1 352 670 A1 ist ein Koaxialschlauchsystem bekannt, bei dem der Heizdraht ebenfalls im Expirationsgas führenden Ringraum des Koaxialsystems angeordnet ist. Der Heizdraht ist von einer isolierenden, hydrophoben Schicht umgeben. Auch dieses Koaxialsystem ist konstruktiv aufwendig.

Die DE 600 20 024 T2 zeigt ein Koaxialbeatmungsschlauchsystem, bei dem sich das Inspirationsgas zusammen mit einem Heizelement in dem äußeren Ringraum des Koaxialschlauchssystems befindet. Dieser äußere Ringraum muss jedoch mit einem zusätzlichen, mit Luft gefüllten Ringraum umgeben sein. Dies führt zu einer aufwändigen Konstruktion.

### Aufgabe

Die Aufgabe der vorliegenden Erfindung besteht darin, ein Beatmungsschlausystem zur Verfügung zu stellen, welches einfach und kostengünstig herzustellen ist und eine auch für Frühchen oder Neugeborenen ausreichende Konditionierung des Atemgases ermöglicht.

### Lösung gemäß der Erfindung

Die vorliegende Aufgabe wird bei dem gattungsgemäßen Schlauchsystem durch die Merkmale des kennzeichnenden Teils von Anspruch 1 gelöst. Am Abzweigstück ist ein Stecker für den Heizdraht vorgesehen. Dies ermöglicht es, den Heizdraht in einfacher Weise am Beatmungsschlauchsystem anzuschließen. Dadurch, dass der Heizdraht für das Beatmungsgas und/oder der Heizdraht für das Expirationsgas innerhalb des ersten Schlauchs bzw. dritten Schlauchs langgestreckt angeordnet ist bzw. sind, wird eine optimale Wärmeübertragung und damit Atemgaskonditionierung erreicht. Die Erfindung eignet sich auf Grund ihres konstruktiven Aufbaus ganz besonders für eine derartige Ausgestaltung des Heizelements.

Als eine einfach zu realisierende Lösung der Steuerung der Inspiration bzw. Expiration kann eine Membran bzw. ein flexibles Plättchen vorgesehen sein, die bzw. das abhängig von der Richtung der Druckbeaufschlagung einen Durchlass des Beatmungsgases sperrt oder freigibt.

Eine entsprechende Membran kann zusätzlich auch im Leitungsweg des Expirationsgases angeordnet sein. Sie vermeidet einen Rückfluß an Expirationsgas und damit auch eine nachteilige Beeinträchtigung der Temperaturkonstanz des Koaxial-Beatmungsschlauchsystems hierdurch.

Besonders vorteilhaft ist es, wenn die Membran für das Inspirationsgas und/oder die Membran für das Expirationsgas im zweiten Anschlussstück bzw. im patientenseitigen Konnektor untergebracht ist bzw. sind. Dementsprechend kann oder können die Membran(en) bereits während der Fertigung des Anschlussstücks in letzteres eingearbeitet werden.

Wenn der Heizdraht für das Beatmungsgas und/oder der Heizdraht für das Expirationsgas innerhalb des ersten Schlauchs bzw. dritten Schlauchs zumindest weitgehend nicht an der Schlauchwand anliegend ohne Montagepunkte entlang des Weges angeordnet ist bzw. sind, wird eine optimale Wärmeübertragung und damit Atemgaskonditionierung erreicht. Die Erfindung eignet sich auf Grund ihres konstruktiven Aufbaus ganz besonders für eine derartige Ausgestaltung des Heizelements. Entsprechendes gilt auch für den Winkelkonnektor.

### Beschreibung der Erfindung anhand eines Beispiels

Eine zweckmäßige Ausgestaltung des erfindungsgemäßen Beatmungsschlauchsystems ist anhand von Zeichnungen näher erläutert. Es zeigen:
- Fig. 1: eine stark vereinfachte schematische Darstellung einer beispielhaften Ausgestaltung des erfindungsgemäßen Beatmungsschlauchsystems einschließlich Beatmungsgerät;
- Fig. 2: eine vergrößerte Darstellung des Beatmungsschlauchsystems nach Fig. 1 sowie
- Fig. 3: eine Darstellung des patientenseitigen Anschlussstücks mit Membran für das Inspirations- sowie Exspirationsgas.

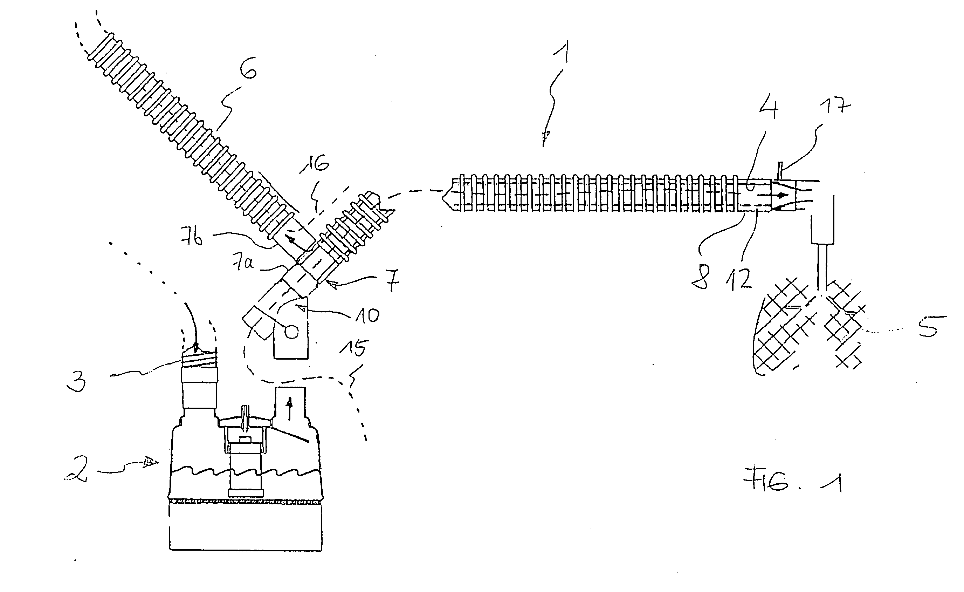

Die Darstellung in Fig. 1 zeigt die für die Beatmung eines Patienten 5 notwendigen Bestandteile. Sie umfassen zum ein sogenanntes Beatmungsgerät 2 oder auch Beatmungsgaskonditioniereinrichtung genannt, die dazu dient, das von einer (nicht dargestellten) Versorgung über eine Zuleitung 3 zur Verfügung gestellte Beatmungsgas auf die gewünschte Temperatur sowie relative Feuchtigkeit zubringen. Üblicherweise wird das Beatmungsgas derart konditioniert, dass es eine Temperatur von 37°C und eine relative Feuchtigkeit von 100% entsprechend 44mg Feuchtigkeit / Liter Beatmungsgas aufweist. Ein entsprechendes Beat mungsgerät ist in der DE 10 2008 024 728 A1, beschrieben.

Zur Verbindung des Beatmungsgeräts 2 mit dem Patienten 5 dient das Beatmungsschlauchsystem 1. Es ist im Falle der vorliegenden Erfindung als Koaxialschlauchsystem konzipiert und umfasst einen ersten, inneren Schlauch 4, mittels welchem das vom Beatmungsgerät 2 konditionierte Beatmungsgas dem Patienten 5 zugeführt wird. Um zu vermeiden, dass bedingt durch äußere Einflüsse die Temperatur des in dem Beatmungsgerät 2 bereit gestellten Beatmungsgases sich ändert, d.h. eine Temperaturerniedrigung während des Transports zum Patienten eintritt, befindet sich im Inneren des ersten Schlauchs 4 eine Heizeinrichtung in Form eines Heizdrahts 15.

Zur Rückführung des vom Patienten 5 ausgeatmeten Gases (Expirationsgas) dient der durch den zweiten Schlauch 8 gebildete Ringraum 12. Das durch den Ringraum 12 bis zu dem Abzweigstück 7 strömende Expirationsgas bewirkt aufgrund des Koaxialsystems zusätzlich ein günstige thermische Beeinträchtigung des Inspirationsgases und sorgt dafür, dass die Temperatur des Inspirationsgases konstant bleibt.

Im Bereich des Abzweigsstücks 7 wird das Expirationsgas über einen dritten Schlauch 6 abgeführt und ggf. zur Triggerung dem Beatmungsgerät 2 wieder zugeführt oder ins Freie ausgeblasen.

Zur Vermeidung einer zu starken Abkühlung des Expirationsgases auf dem Weg zum Beatmungsgerät 2 ist auch im Bereich des dritten Schlauchs 6 eine Heizeinrichtung z.B. in Form eines Heizdrahtes 16 vorgesehen, damit kein Kondensat in das Beatmungsgerät 2 eindringen kann

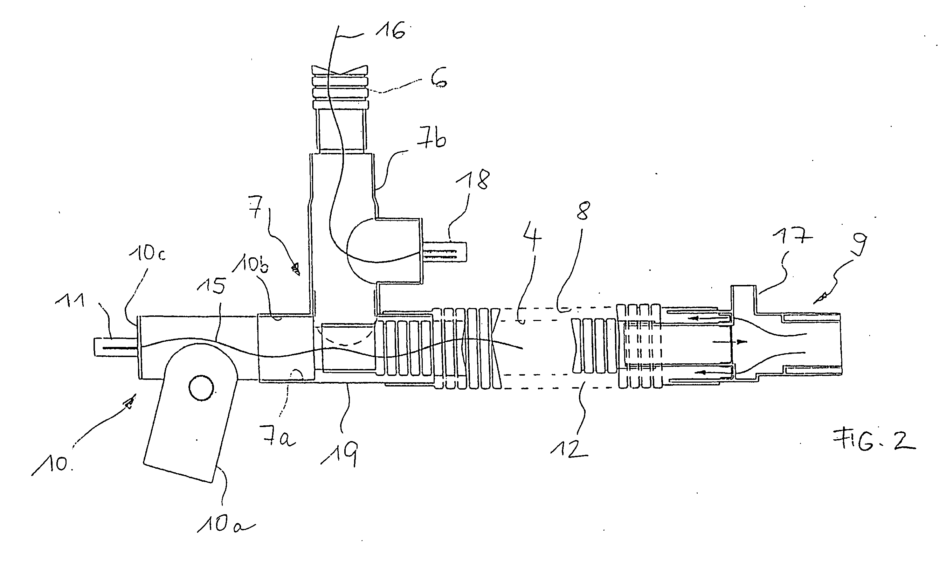

Das Abzweigstück 7, welches zweckmäßigerweise als T-Stück ausgebildet ist, steht vorzugsweise unmittelbar mit einem Winkelkonnektor 10 in Verbindung, mit dem eine Verbindung des erfindungsgemäßen Schlauchsystems mit dem Beatmungsgerät 2 erfolgt. Wie aus Figur 2 ersichtlich verläuft der Heizdraht 15 bis auf endständige Befestigungspunkte entlang seiner Erstreckung ohne Fixierungspunkte, nahezu lose und weitgehend gestreckt innerhalb des inneren Schlauchs 4. Er ermöglicht demzufolge eine sehr gute Wärmeübertragung entlang seines kompletten im Querschnitt betrachteten Umfangs auf das Beatmungsgas. Erfindungsgemäß erstreckt sich der Heizdraht 15 vollständig durch den lang gestreckten Teil (zweiter Teil 7a) des Abzweigstücks 7 hindurch bis zu dem stirnseitigen Ende des 10c des Winkelkonnektors 10. Im Bereich stirnseitigen Endes 10c kann, wie in Figur 2 dargestellt, ein Stecker 11 zur einfachen Kontaktierung des Heizdrahts 15 vorgesehen sein. Auf der dem Stecker 11 gegenüberliegenden Seite des Beatmungsschlauchsystems 1 ist der Heizdraht 15 in geeigneter Weise fixiert (nicht dargestellt), so dass er in der vorbeschriebenen Art und Weise innerhalb des ersten Schlauchs 4 nahezu frei schwebend positioniert ist.

Der Winkelkonnektor 10 besitzt ein, vorzugsweise drehbar gelagertes, Anschlussstück 10a zur Verbindung mit dem Beatmungsgerät 2.

Im Bereich des Abzweigstücks 7 ist ebenfalls ein Stecker 18 den Heizdraht 16 vorgesehen. Der Stecker 18 befindet sich in einem Vorsprung des abzweigenden Teils (erster Teil 7b) des Abzweigstücks 7. Der Winkelkonnektor 10 sowie das insbesondere T-förmig ausgebildete Abzweigstück 7 liegen unmittelbar aneinander. Zweckmäßigerweise können diese beiden Bauteile im Form eines einzigen Bauteils vorgesehen sein.

Patientenseitig ist ein Konnektor 9 vorgesehen, welcher mit einer entsprechenden Applikationseinrichtung (Tubus, Atemmaske oder Nasenbrille) verbunden wird. Der Konnektor 9 kann zudem ein Anschluss 17 für Medikamentenzufuhr und/oder für einen (nicht dargestellten) Temperatursensor beinhalten.

Wie aus Fig. 2 ersichtlich strömt Beatmungsgas mittig in den Konnektor 9 ein, wohingegen Expirationsgas außenseitig in den Ringspalt 12 eintritt und von dort zum Abzweigstück 7 zurück strömt.

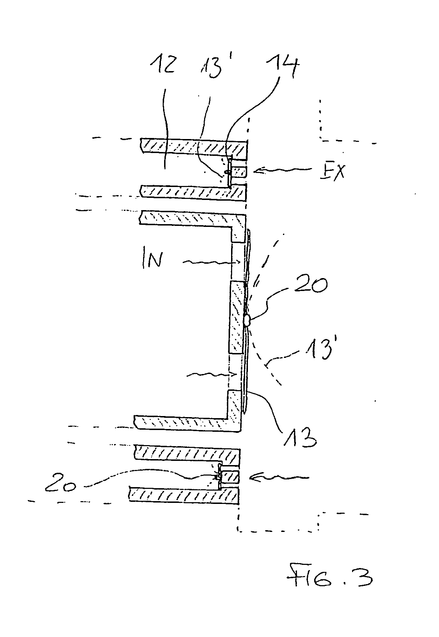

Wie aus Fig. 3, welche einen Teilbereich des patientenseitigen Konnektors 9 zeigt, deutlich wird, wird ein Rückfluss des Beatmungsgases zurück in dem Bereich des ersten Schlauchs 4 hinein durch eine Membran 13 bzw. durch ein flexibles Plättchen verhindert. Wie durch die strichlierte Linie und die Bezugsziffer 13' dargestellt öffnet sich, d.h. wölbt sich die Membran 13 bei erhöhtem Druck, wohingegen sich die Membran an den Konnektor 9 flach anlegt, falls ein aus der anderen Richtung erfolgender Strom an Expirationsgas einsetzt.

In gleicher Weise arbeitet auch die Membran 14 im Bereich der Zuleitung zum Ringraum 12. Die jeweilige Membran 13 bzw. 14 ist vorzugsweise in ihrer Mitte durch ein geeignetes Befestigungselement 19 bzw. 20 fixiert.

Die Membransteuerung stellt eine konstruktiv einfache Möglichkeit der Realisierung dar.

Das erfindungsgemäße Beatmungsschlauchsystem ist insbesondere als Einwegbeatmungsschlauchsystem konzipiert. Es eignet sich vor allem für den Einsatz bei der Beatmung von Neugeborenen oder Frühchen.

### BEZUGSZEICHENLISTE

- 1: Beatmungsschlauchsystem
- 2: Beatmungsgerät
- 3: Zuleitung
- 4: erster Schlauch
- 5: Patient
- 6: dritter Schlauch
- 7: Abzweigstück
- 8: zweiter Schlauch
- 9: patientenseitiger Konnektor
- 10: Winkelkonnektor
- 11: Stecker
- 12: Ringkanal
- 13: Membran
- 14: Membran
- 15: Heizdraht Inspiration
- 16: Heizdraht Exspiration
- 17: Anschluss für Medikamentenzufuhr
- 18: Stecker
- 19: Befestigungselement
- 20: Befestigungselement

## Patentansprüche

1. Beatmungsschlauchsystem, vorzugsweise Einweg-Beatmungsschlauchsystem (1) insbesondere für Neugeborene, Säuglinge oder Kleinkinder zur Beatmung mit temperierter und/oder angefeuchteter Beatmungsluft sowie zur Rückführung von Expirationsgas mit
einem ersten Anschlussstück zum Anschluss des Beatmungsschlauchsystems (1) an ein Beatmungsgerät (2),
einem zweiten Anschlussstück zum Anschluss des Beatmungsschlauchsystems (1) an eine Applikationseinrichtung am Patienten,
einem ersten Schlauch (4), welcher Beatmungsgas vom Beatmungsgerät (2) zum Patienten leitet,
einem zweiten Schlauch (8), welcher Expirationsgas vom Patienten wegleitet, wobei
der erste Schlauch (4) zumindest über einen Teil seiner Gesamtlänge innerhalb des zweiten Schlauchs (8) verläuft und
ein Heizdraht (15) für das Beatmungsgas vorgesehen ist,
der Heizdraht (15) innerhalb des ersten Schlauchs (4) verläuft,
am vom Patienten abgewandten Ende des zweiten Schlauchs (8) ein T- förmiges Abzeigstück (7) mit einem ersten Teil (7b), an dem ein dritter Schlauch (6) für das Expirationsgas angrenzt, sowie einem zweiten Teil (7a) für das Beatmungsgas vorgesehen ist,
der Heizdraht (15) den zweiten Teil (7a) des Abzweigstücks (7) vollständig durchläuft,
in einem dritten Schlauch (6) für das Expirationsgas ebenfalls ein Heizdraht (16) verläuft,
das erste Anschlussstück als Winkelverbinder (10) ausgebildet ist,
der Winkelverbinder (10) über einen Anschluss (10a) für das Beatmungsgerät (2) sowie über einen weiteren Anschluss (10b) für das Abzweigstück (19) verfügt,
**dadurch gekennzeichnet, dass**
ein Stecker (11) für den Anschluss des Heizdrahts (15) an dem dem weiteren Anschluss (10b) gegenüber liegenden Ende (10c) des Winkelverbinders (10) positioniert ist,
ein weiterer Stecker (18) für den Anschluss des Heizdrahts (16) am ersten Teil (7b) des Abzweigstücks (7) positioniert ist, und
der Heizdraht (15) und/oder der Heizdraht (16) innerhalb des ersten Schlauchs (4) bzw. dritten Schlauchs (6) langgestreckt angeordnet ist.

2. Beatmungsschlauchsystem nach Anspruch 1,
**dadurch gekennzeichnet, dass**
eine Membran (13) vorgesehen ist, die abhängig von der Richtung der Druckbeaufschlagung einen Durchlass des Beatmungsgases sperrt oder freigibt.

3. Beatmungsschlauchsystem nach mindestens einem der vorangegangenen Ansprüche,
**dadurch gekennzeichnet, dass**
eine Membran (14) vorgesehen ist, die abhängig von der Richtung der Druckbeaufschlagung einen Durchlass des Exspirationsgases sperrt oder freigibt.

4. Beatmungsschlauchsystem nach Anspruch 2 oder 3,
**dadurch gekennzeichnet, dass**
die Membran (13) und/oder die Membran (14) in einem patientenseitigen Konnektor (9) untergebracht ist bzw. sind.

5. Beatmungsschlauchsystem nach mindestens einem der vorangegangenen Ansprüche,
**dadurch gekennzeichnet, dass**
der Heizdraht (15) und/oder der Heizdraht (16) zumindest weitgehend nicht an der Schlauchwand anliegend ohne Montagepunkte entlang des Weges angeordnet ist.

## Claims

1. Breathing tube system, preferably a disposable breathing tube system (1), particularly for neonates, babies or young children, for respiration with temperature-controlled and/or humidified respiratory air and for the return of expiratory gas, with
a first attachment piece for attaching the breathing tube system (1) to a respirator (2),
a second attachment piece for attaching the breathing tube system (1) to an applicator device on the patient,
a first tube (4), which conveys respiratory gas from the respirator (2) to the patient,
a second tube (8), which conveys expiratory gas away from the patient, wherein
the first tube (4) runs, at least over part of its overall length, inside the second tube (8), and
a heating wire (15) for the respiratory gas is provided,
the heating wire (15) runs inside the first tube (4),
a T-shaped branch piece (7), provided at the end of the second tube (8) directed away from the patient, has a first part (7b), which is adjoined by a third tube (6) for the expiratory gas, and a second part (7a) for the respiratory gas,
the heating wire (15) runs completely through the second part (7a) of the branch piece (7),
a heating wire (16) likewise runs inside a third tube (6) for the expiratory gas,
the first attachment piece is designed as an angle connector (10),
the angle connector (10) has an attachment (10a) for the respirator (2) and a further attachment (10b) for the branch piece (19),
**characterized in that**
a plug (11) for attachment of the heating wire (15) is positioned on that end (10c) of the angle connector (10) lying opposite the further attachment (10b),
a further plug (18) for attachment of the heating wire (16) is positioned on the first part (7b) of the branch piece (7), and
the heating wire (15) and/or the heating wire (16) is arranged in an elongate form inside the first tube (4) and/or third tube (6).

2. Breathing tube system according to Claim 1, **characterized in that** a membrane (13) is provided, which blocks or frees a passage of the respiratory gas depending on the direction of the applied pressure.

3. Breathing tube system according to at least one of the preceding claims, **characterized in that** a membrane (14) is provided, which blocks or frees a passage of the expiratory gas depending on the direction of the applied pressure.

4. Breathing tube system according to Claim 2 or 3, **characterized in that** the membrane (13) and/or the membrane (14) is/are accommodated in a patient-side connector (9).

5. Breathing tube system according to at least one of the preceding claims, **characterized in that** the heating wire (15) and/or the heating wire (16) is arranged at least substantially not bearing on the tube wall and without mounting points along the path.

## Revendications

1. Système de tube respiratoire, de préférence système de tube respiratoire jetable (1) en particulier pour nouveaux-nés, nourrissons ou petits enfants, pour la respiration avec de l'air respiratoire régulé en température et/ou humidifié ainsi que pour la recirculation de gaz expiratoire, comprenant
un premier élément de raccordement pour le raccordement du système de tube respiratoire (1) à un appareil de respiration (2),
un deuxième élément de raccordement pour le raccordement du système de tube respiratoire (1) à un appareil d'application sur le patient,
un premier tube flexible (4), qui conduit du gaz respiratoire depuis l'appareil de respiration (2) jusqu'au patient,
un deuxième tube flexible (8) qui conduit du gaz expiratoire depuis le patient,
le premier tube flexible (4) s'étendant au moins sur une partie de toute sa longueur à l'intérieur du deuxième tube flexible (8) et
un filament chauffant (15) pour le gaz respiratoire étant prévu,
le filament chauffant (15) s'étendant à l'intérieur du premier tube flexible (4),
à l'extrémité du deuxième tube flexible (8) éloignée du patient, un élément de dérivation (7) en forme de T avec une première partie (7b) au niveau de laquelle un troisième tube flexible (6) pour le gaz expiratoire est raccordé ainsi qu'une deuxième partie (7a) pour le gaz respiratoire étant prévu,
le filament chauffant (15) traversant complètement la deuxième partie (7a) de l'élément de dérivation (7),
un filament chauffant (16) s'étendant également dans un troisième tube flexible (6) pour le gaz expiratoire,
le premier élément de raccordement étant réalisé sous forme de connecteur coudé (10),
le connecteur coudé (10) disposant d'un raccord (10a) pour l'appareil de respiration (2) ainsi que d'un raccord supplémentaire (10b) pour l'élément de dérivation (19),
**caractérisé en ce**
**qu'**une fiche (11) pour le raccordement du filament chauffant (15) est positionnée à l'extrémité (10c) du connecteur coudé (10) éloignée du raccord supplémentaire (10b),
une fiche supplémentaire (18) pour le raccordement du filament chauffant (16) est positionnée au niveau de la première partie (7b) de l'élément de dérivation (7), et
le filament chauffant (15) et/ou le filament chauffant (16) sont disposés de manière étirée en longueur à l'intérieur du premier tube flexible (4), respectivement du troisième tube flexible (6).

2. Système de tube respiratoire selon la revendication 1,
**caractérisé en ce**
**qu'**une membrane (13) est prévue, laquelle bloque ou libère un passage de gaz respiratoire en fonction de la direction de la sollicitation par pression.

3. Système de tube respiratoire selon au moins l'une quelconque des revendications précédentes,
**caractérisé en ce**
**qu'**une membrane (14) est prévue, laquelle bloque ou libère un passage de gaz expiratoire en fonction de la direction de la sollicitation par pression.

4. Système de tube respiratoire selon la revendication 2 ou 3,
**caractérisé en ce que**
la membrane (13) et/ou la membrane (14) est ou sont montée(s) dans un connecteur (9) du côté du patient.

5. Système de tube respiratoire selon au moins l'une quelconque des revendications précédentes,
**caractérisé en ce que**
le filament chauffant (15) et/ou le filament chauffant (16) sont disposés sur leur chemin au moins essentiellement sans s'appliquer contre la paroi du tube flexible et sans points de montage.
